# EUROPEAN PATENT SPECIFICATION

(11) **EP 1 001 773 B1**
(45) Date of publication and mention of the grant of the patent: **06.02.2002**
(21) Application number: 98935235.6
(22) Date of filing: 06.08.1998
(51) Int. Cl.: A61K 31/455, A61K 9/20

(54) **ORAL SOLID PHARMACEUTICAL COMPOSITIONS CONTAINING NICORANDIL FOR A MODULATED RELEASE AND THE PROCESS FOR THEIR PREPARATION**
ORAL ANZUWENDENDE FESTE ARZNEIZUBEREITUNGEN MIT MODULIERTER WIRKSTOFFABGABE ENTHALTEND NICORANDIL UND VERFAHREN ZU DEREN HERSTELLUNG
COMPOSITIONS PHARMACEUTIQUES SOLIDES POUR ADMINISTRATION PAR VOIE ORALE PERMETTANT LA LIBERATION MODULEE DE NICORANDIL ET LEUR PROCEDE DE PREPARATION

(30) Priority: 08.08.1997 IT MI971913
(43) Date of publication of application: 24.05.2000
(73) Proprietor: Biomedica Foscama Industria Chimico-Farmaceutica S.p.A., 03013 Ferentino (IT)
(72) Inventor: SACCUCCI, Giampaolo, I-03036 Isola del Liri (IT); GIOVANNONE, Daniele, I-03100 Frosinone (IT); CARLI, Fabio, I-34136 Trieste (IT)
(74) Representative: Savi, Alberto
(86) International application number: IB9801207
(87) International publication number: WO9907370

(56) References cited:
- EP-A- 0 167 825
- EP-A- 0 185 347
- EP-A- 0 230 932
- EP-A- 0 598 337
- GB-A- 2 194 147
- GB-A- 2 290 965
- CHEMICAL ABSTRACTS, vol. 123, no. 24, 11 December 1995 Columbus, Ohio, US; abstract no. 322120, XP002081399 cited in the application & JP 07 228531 A (TOYO CAPSEL KK,JP) 29 August 1995
- CHEMICAL ABSTRACTS, vol. 125, no. 14, 30 September 1996 Columbus, Ohio, US; abstract no. 177474, XP002081400 & JP 08 175996 A (TAIYO PHARMA IND.,JP) 9 July 1996
- CHEMICAL ABSTRACTS, vol. 129, Columbus, Ohio, US; abstract no. 193510, XP002081401 & JP 10 203935 A (SANSEI PHARM. CO. LTD.,JP) 4 August 1998

## Description

This invention refers to new pharmaceutical compositions in a solid form for the modulated release of Nicorandil (N-(2-hydroxyethyl) nicotinamide nitric ester), their utilization in preparing drugs for oral use, and a process for preparing said compositions.

In particular, this invention comprises oral pharmaceutical compositions in a solid form, for the modulated release of Nicorandil as an active ingredient, stabilised by the presence of a glyceride of a saturated fatty acid having a number of carbon atoms equal to or greater than 18 as a pharmaceutically acceptable excipient.

Nicorandil is a coronary vasodilator endowed with a complex pharmacological activity, being both an organic nitrate and a potassium channel activator. It is used for the therapy of a number of cardiovascular diseases such as myocardial ischemia (especially angina pectoris) and congestive heart failure. The oral drugs containing pharmaceutical compositions of Nicorandil in a solid form suitable for repeated administrations are particularly used in therapy for preventing anginose attacks.

The pharmaceutical compositions of Nicorandil in a solid form are generally characterised by their unsatisfactory stability, especially in the presence of moisture and such other factors as acidity, temperature, light and oxygen. Particular precautions are therefore required throughout their manufacturing process and during storage, to avoid the products' contact with moisture and to inhibit other degradation processes which may cause an appreciable reduction of the active ingredient content.

The stability of the pharmaceutical compositions of Nicorandil in a solid form is further impaired when they are subjected to a certain degree of compression, which may alter the crystalline structure of the active ingredient.

It is therefore necessary to effect a proper choice of the excipients for the pharmaceutical compositions, so as to achieve a tablet formulation endowed with an adequate hardness, while applying a reduced of compressing strenght. The European Patent EP 230,932 describes a method for preparing parmaceutical compositions of Nicorandil containing higher saturated aliphatic fatty acids or alcohols, which may also include fumaric, oxalic, salicylic, tartaric and/or glutaric acids.

The European Patent EP 185,347 describes pharmaceutical compositions of Nicorandil containing a sugar with a particle size not exceeding 10 µm and/or an organic acid.

On the other hand, the Japanese Patent Application JP 07.228.531 relates to capsules of Nicorandil containing an oil base (for ex. corn oil) and an aqueous base (for ex. polyethyleneglycol). However, in the pharmaceutical compositions of Nicorandil in a solid form currently now available the stability of the active ingredient is still unsatisfactory. In addition, their pharmacokinetic profile is far from being the most suitable, since an immediate release of the active ingredient occurs, leading to a rapid build-up of high blood concentrations of Nicorandil, which are unnecessary from a therapeutical point of a view, while causing a high incidence of side effects, firstly headaches and secondly postural hypotension. In this regard, it is worth mentioning that these effects are a frequent reason for suspending the treatment, while hindering the administration of higher dosages, which may be needed for therapeutic purposes.

Surprisingly, it has now been discovered that it is possible to favourably achieve an oral pharmaceutical composition of Nicorandil in a stable solid form, by combining it, with a glyceride of a saturated fatty acid having a number of carbon atoms equal to or greater than 18 as an eccipient. Moreover, the addition of such excipient allows reducing the compressive strenght needed for tableting, thus preserving the integrity of the microcrystalline structure of the active ingredient and ensuring a longer stability of the same. It is worth noting the unexpected circumstance that the pharmaceutical compositions of this invention have been stabilised without adding an organic acid, despite the known fact that the stability of the active ingredient is enhanced in an acid environment. Moreover, the tablets achieved by the process referred to in this invention are capable of releasing the active ingredient at a slower rate, thus ensuring its favourable absorption and a good bioavailability while preventing the occurrence of high plasma concentration, with the resulting advantages in terms of a lesser frequency and severity of the side effects mentioned above.

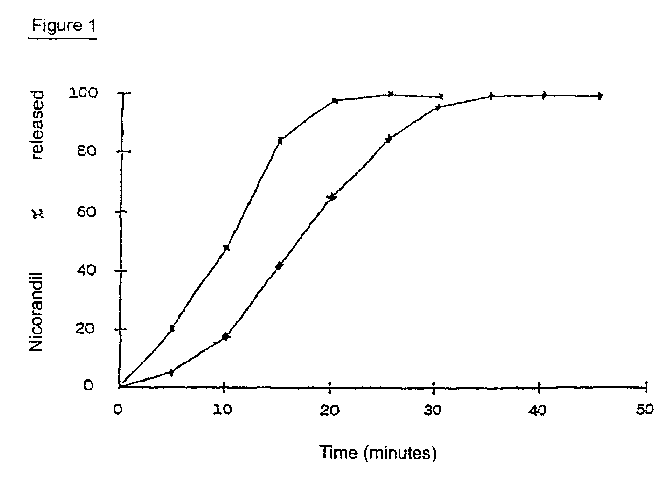

The graph on Figure 1 shows the dissolving profiles of a tablet formulated as described in the example (---◆---) and a commercial tablet containing stearic acid (---n---)(whose composition is reported in the comparative example 1), commonly used in therapy. The dissolution test was carried out in a 500 ml volume of a buffered solution at a pH of 1.1 at 37°C, by using an apparatus equipped with stirring paddles rotating at 120 rpm.

### (Figure 1)

Table 1 compares the most significant parameters drawn from the dissolution profiles of the composition of this invention and the commercial tablet.

**Table I.**

| | Dissolving time t ½ | Releasing speed (*) |
|---|---|---|
| Tablet in the example | 16.8 ± 0.8 | 0.46 |
| Commercial tablet | 10.2 ± 0.5 | 0.64 |

| | | |
|---|---|---|
| (*) calculated in the releasing interval of 20% to 80%. | | |

If one considers that the absorption of orally administered Nicorandil (in conventional tablets) is extremely rapid, resulting in a T_{max.} of 30 minutes (Am.J.Cardiol. 1989, 63, 25J-33J), it can be easily understood that the significant increase of the dissolution time in vitro and the slower release rate observed with the composition of this invention may lead to a marked improvement of the pharmacokinetic absorption parameters, and in particular to an increase of the T max and a corresponding lowering of the plasma concentration peak, with the resulting benefits in terms of a lower frequency and severity of side effects. One object of this invention is therefore a solid pharmaceutical composition of Nicorandil with a modulated release, containing an appropriate quantity of a glyceride of a saturated fatty acid having a number of carbon atoms equal to or greater than 18 as a pharmaceutically acceptable excipient.

The pharmaceutical compositions of this invention may contain an appropriate quantity of crystalline Nicorandil in a mixture containing at least 1% of a glyceride of a saturated fatty acid having a number of carbon atoms equal or greater than 18. In particular, glyceril behenate is used as a glyceride of saturated fatty acid.

Another object of this invention is a process for preparing a solid pharmaceutical composition with a modulated release of Nicorandil, which is admixed with the said glyceride of a saturated fatty acid and optionally with any pharmaceutically compatible diluents, disgregants, lubricants, dyes and/or binders, each in the quantities commonly employed for pharmaceutical preparations.

In these compositions both the active ingredient and the diluents, disgregants, lubricants, dyes and/or binders are present in the quantities commonly employed and known in the literature for preparing oral drugs, while the glyceride of a saturated fatty acid is added to said compositions in a quantity of not less than 1% by weight with respect to the composition's total weight.

In one preferred embodiment of this invention the excipients are added to the active ingredient by successive dilutions and mixing under dry conditions. The tablets are produced by direct compression of the powder mixture thus achieved.

Alternatively, Nicorandil may be formulated by using the excipients described in this invention in the form of granules, capsules or enteric granules.

Another object of this invention is the use of said pharmaceutical compositions for the preparation of drugs to be administered orally.

The following example is presented in order to better illustrate the invention, without however limiting it. The comparative example 1 provides the composition of commercial Nicorandil tablets containing an organic acid (stearic acid), while the comparative example 2 refers to unstabilised Nicorandil tablets, free from organic acid.

### Example

| Formulation in tablets (weight per tablet): | |
|---|---|
| Nicorandil | 10.0 mg |
| Atomised glyceril behenate | 10.2 mg |
| Sorbitol | 67.9 mg |
| Microcrystalline cellulose | 3.2 mg |
| Croscarmellose sodium | 5.0 mg |
| Talc | 2.5 mg |
| Magnesium stearate | 1.2 mg |
| Total | 100 mg |

20.0 mg of crystalline Nicorandil and 20.4 g of atomised glyceril behenate were mixed for about an hour in a biconical mixer. 135.8 g of sorbitol (having a particle size of 300 µm) were then added to the mixture and again mixed for one hour. The resulting mixture was then added with 6.4 g of microcrystalline cellulose and 10.0 g of croscarmellose sodium, in this order, by mixing for 10 minutes after each addition. 5.0 g of talc and 2.4 g of magnesium stearate were finally added and mixed for another hour, thus obtaining the powder to be tableted. The compression was carried out by using an alternative KORSH EK O tableting machine equipped with a punch of 6 mm diameter, to obtain tablets of 100 mg each.

### Comparative example 1

| Formulation in tablets (weight per tablet): | |
|---|---|
| Nicorandil | 10 mg |
| Mannitol | 76 mg |
| Corn starch | 1 mg |
| Sodium carboxymethylcellulose | 5 mg |
| Stearic acid | 8 mg |
| Total | 100 mg |

### Comparative example 2

| Formulation in tablet (weight per tablet): | |
|---|---|
| Nicorandil | 10.0 mg |
| Mannitol | 72.5 mg |
| Glycyrrhizic acid, ammonium salt | 8.0 mg |
| Croscarmellose Sodium | 4.8 mg |
| Corn starch | 1 mg |
| Talc | 2.5 mg |
| Magnesium stearate | 1.2 mg |
| Total | 100 mg |

36.5 of mannitol, 2.41 g of croscarmellose sodium and 0.48 g of corn starch were mixed for 40 minutes in a biconical mixer. The resulting mix was then treated with 10 mg water. The mix was passed through a 2 mm mesh sieve and then dried in a fluidized bed drier at 40°C for 2 hours. The dried granulating was sieved through successive screens having a mesh size of 1.00 mm and 0.63 mm, respectively. The granular mass thus obtained was treated with 5.00 g of Nicorandil and 4.00 g. of glycyrrhizic acid ammonium, salt and mixed for 2 hours 1.2 g of talcum and 0.60 g of magnesium stearate were finally added to obtain the mixture ready for tableting. The compression was carried out by using an alternative KORSH EK O tableting machine equipped with a punch of 6 mm diameter, to obtain tablets of 100 mg each.

Table II shows the results of the stability tests run under accelerated conditions (50°C in a thermostatically controlled oven in the presence of silica gel as a desiccant) on the composition of this invention, while comparing it with samples of the composition listed in the comparative example 1 (commercial composition), stored under the same conditions. The table shows both the active ingredient and the inorganic nitrate content (the former expressed as % of the concentration at the time 0, the latter in mg/g).

**Table II.**

| Time (days) | 0 | 7 | 14 | 12 |
|---|---|---|---|---|
| Composition of the invention (% Nicorandil) | 100 | 93.8 | 55.1 | 30.6 |
| Composition of the comparative example 1 (% of Nicorandil) | 100 | 84.2 | 59.3 | 14.8 |
| Composition of the invention (mg/g N03) | 0 | 8.3 | 42.9 | 67.6 |
| Composition of the comparative example (mg/g N03) | 0 | 17.9 | 38.4 | 78.1 |

Table III shows the results of the stability tests under accelerated conditions (40°C in a thermostatically controlled oven in the presence of silica gel as a desiccant) of the composition of the invention in comparison with samples of the composition of the comparative example 2, stored under the same conditions. It shows both the active ingredient and the inorganic nitrate content (the former expressed as % of the concentration at the time 0, the latter in mg/g).

**Table III.**

| Time (days) | 0 | 7 | 16 | 30 | 47 |
|---|---|---|---|---|---|
| Composition of the invention (% Nicorandil) | 100 | 98.7 | 96.3 | 91.2 | 85.7 |
| Composition of the comparative example 1 (% of Nicorandil) | 100 | 96.8 | 93.9 | 85.4 | 60.8 |
| Composition of the invention (mg/g N03) | 0 | 0 | 2.7 | 6.3 | 15.1 |
| Composition of the comparative example (mg/g N03) | 0 | 3.2 | 4.7 | 12.8 | 40.6 |

## Claims

1. An oral pharmaceutical composition in a solid form for a modulated release of Nicorandil, **characterised by** the fact that it contains an appropriate quantity of a glyceride of a saturated aliphatic fatty acid with a number of carbon atoms equal or greater than 18, as a pharmaceutically acceptable excipient.

2. A pharmaceutical composition according to the Claim 1, **characterised by** the fact that said glyceride of a saturated fatty acid is present in a quantity of not less than 1% by weight with respect to the total weight of the composition.

3. A pharmaceutical composition according to the Claims 1 and 2, **characterised by** the fact that the glyceride of a saturated fatty acid is glyceril behenate.

4. A process for preparing a pharmaceutical composition in accordance with one of the claims from 1 to 3, **characterised by** the fact that the active ingredient Nicorandil is mixed with a glyceride of a saturated fatty acid with a number of carbon atoms equal to or greater than 18 and optionally with appropriate and pharmaceutically compatible diluents, disgregants, lubricants, dyes and/or binders, each in the quantities appropriate for pharmaceutical compositions.

5. A process in accordance with the Claim 4, **characterised by** the fact that the excipients are added to the active ingredient by successive dilutions and mixing under dry conditions.

6. The use of a pharmaceutical composition in accordance with the claims from 1 to 3, for the preparation of a drug suitable for oral administration.

## Patentansprüche

1. Orale pharmazeutische Zusammensetzung in fester Form für eine gesteuerte Freisetzung von Nicorandil, **dadurch gekennzeichnet, daß** sie eine geeignete Menge eines Glycerids einer gesättigten aliphatischen Fettsäure mit einer Anzahl an Kohlenstoffatomen von gleich oder mehr als 18 als pharmazeutisch annehmbaren Exzipienten enthält.

2. Pharmazeutische Zusammensetzung gemäß Anspruch 1, **dadurch gekennzeichnet, daß** das Glycerid der gesättigten Fettsäure in einer Menge von nicht geringer als 1 Gew.%, bezogen auf das Gesamtgewicht der Zusammensetzung, vorhanden ist.

3. Pharmazeutische Zusammensetzung gemäß den Ansprüchen 1 und 2, **dadurch gekennzeichnet, daß** das Glycerid der gesättigten Fettsäure Glycerylbehenat ist.

4. Verfahren zur Herstellung einer pharmazeutischen Zusammensetzung gemäß einem der Ansprüche 1 bis 3, **dadurch gekennzeichnet, daß** der aktive Bestandteil Nicorandil mit einem Glycerid einer gesättigten Fettsäure mit einer Anzahl an Kohlenstoffatomen von gleich oder mehr als 18 und gegebenenfalls mit geeigneten und pharmazeutisch verträglichen Verbindungsmitteln, Zerfallsmitteln, Gleitmitteln, Farbstoffen und/oder Bindemitteln jeweils in den für die pharmazeutische Zusammensetzung geeigneten Mengen vermischt wird.

5. Verfahren gemäß Anspruch 4, **dadurch gekennzeichnet, daß** die Exzipientien zu den aktiven Bestandteilen durch sukzessive Verdünnungen und Mischen unter trockenen Bedingungen zugesetzt werden.

6. Verwendung einer pharmazeutischen Zusammensetzung gemäß den Ansprüchen 1 bis 3 für die Herstellung eines Arzneimittels, das für die orale Verabreichung geeignet ist.

## Revendications

1. Composition pharmaceutique pour une administration par voie orale sous forme solide pour une libération modulée de Nicorandil, **caractérisée par le fait qu'**elle contient une quantité appropriée d'un glycéride d'un acide gras aliphatique sature ayant un nombre d'atomes de carbone supérieur ou égal à 18, en tant qu'excipient acceptable au plan pharmaceutique.

2. Composition pharmaceutique selon la revendication 1, **caractérisée par le fait que** ledit glycéride d'un acide gras saturé est présent en une quantité non inférieure à 1 % en poids par rapport au poids total de la composition.

3. Composition pharmaceutique selon les revendications 1 et 2, **caractérisée par le fait que** ledit glycéride d'un acide gras saturé est le béhénate de glycérile.

4. Procédé de préparation d'une composition pharmaceutique selon l'une des. revendications 1 à 3, **caractérisé par le fait que** l'ingrédient actif Nicorandil est mélangé avec un glycéride d'un acide gras saturé ayant une nombre d'atomes de carbone supérieur ou égal à 18 et éventuellement avec des diluants, délitants, lubrifiants, colorants et/ou liants appropriés et compatibles au plan pharmaceutique, chacun en des quantités appropriées pour des compositions pharmaceutiques.

5. Procédé selon la revendication 4, **caractérisé par le fait que** les excipients sont ajoutés à l'ingrédient actif par dilutions successives et par mélange dans des conditions anhydres.

6. Utilisation d'une composition pharmaceutique selon les revendications 1 à 3, pour la préparation d'un médicament adapté pour une administration par voie orale.
